# EUROPEAN PATENT SPECIFICATION

(11) **EP 3 318 258 B1**
(45) Date of publication and mention of the grant of the patent: **13.05.2020**
(21) Application number: 17189252.4
(22) Date of filing: 14.03.2014
(51) Int. Cl.: A61K 31/454, A61K 31/4985, A61K 31/7056, A61K 45/06, A61P 31/22

(54) **COMBINATION OF DIRECT ACTING ANTIVIRAL AGENTS AND RIBAVIRIN FOR TREATING HCV PATIENTS**
KOMBINATION AUS DIREKTWIRKENDEN ANTIVIRALEN MITTELN UND RIBAVIRIN ZUR BEHANDLUNG VON HCV-PATIENTEN
COMBINAISON D'AGENTS ANTIVIRAUX À ACTION DIRECTE ET DE RIBAVIRINE POUR TRAITER DES PATIENTS INFECTÉS PAR LE HCV

(30) Priority: 14.03.2013 US 201361783437 P
(43) Date of publication of application: 09.05.2018
(62) Divisional of application: 14725812.3
(73) Proprietor: AbbVie Inc., North Chicago, IL 60064 (US)
(72) Inventor: BERNSTEIN, Barry M., Mequon, WI 53092 (US); DUTTA, Sandeep, Lincolnshire, IL 60069 (US); LIU, Wei, Mundelein, IL 60060 (US); PODSADECKI, Thomas J., Northbrook, IL 60062 (US); CAMPBELL, Andrew L., Lake Forest, IL 60045 (US); MENON, Rajeev M., Buffalo Grove, IL 60089 (US); LIN, Chih-Wei, Vernon Hills, IL 60061 (US); WANG, Tianli, Lake Bluff, IL 60044 (US); AWNI, Walid M., Green Oaks, IL 60048 (US); MENSING, Sven, 68305 Mannheim (DE)
(74) Representative: Mewburn Ellis LLP

(56) References cited:
- US-A1- 2012 004 196
- US-A1- 2012 070 416

## Description

### FIELD OF THE INVENTION

The present invention relates to interferon-free treatment for hepatitis C virus (HCV).

### BACKGROUND OF THE INVENTION

The HCV is an RNA virus belonging to the Hepacivirus genus in the Flaviviridae family. The enveloped HCV virion contains a positive stranded RNA genome encoding all known virus-specific proteins in a single, uninterrupted, open reading frame. The open reading frame comprises approximately 9500 nucleotides and encodes a single large polyprotein of about 3000 amino acids. The polyprotein comprises a core protein, envelope proteins E1 and E2, a membrane bound protein p7, and the non-structural proteins NS2, NS3, NS4A, NS4B, NS5A and NS5B.

Chronic HCV infection is associated with progressive liver pathology, including cirrhosis and hepatocellular carcinoma. Chronic hepatitis C may be treated with peginterferon-alpha in combination with ribavirin. Substantial limitations to efficacy and tolerability remain as many users suffer from side effects, and viral elimination from the body is often incomplete. Therefore, there is a need for new therapies to treat HCV infection.

US2012/070416 discloses compounds or pharmaceutically acceptable salts, esters, or prodrugs thereof which inhibit serine protease activity, particularly the activity of hepatitis C virus (HCV) NS3-NS4A protease. Consequently, these compounds interfere with the life cycle of the hepatitis C virus and are useful as antiviral agents. US 2012/004196 concerns compounds effective in inhibiting replication of Hepatitis C virus ("HCV"). This publication also relates to processes of making such compounds, compositions comprising such compounds, and methods of using such compounds to treat HCV infection.

### BRIEF SUMMARY OF THE INVENTION

The present invention provides a combination of at least two direct acting antiviral agents (DAAs) for use in a method for treatment of hepatitis C virus infection, said method comprising administering to a patient in need thereof ribavirin and an effective amount of said combination of at least two DAAs, wherein said treatment does not include administration of interferon to said patient, and said treatment lasts for 12 or 16 weeks, and wherein said at least two DAAs comprises Compound 1, or a pharmaceutically acceptable salt thereof, and Compound 2, or a pharmaceutically acceptable salt thereof;
wherein the method also comprises administration of a DAA which is a HCV polymerase inhibitor, or a pharmaceutically acceptable salt thereof,
wherein Compound 1 is 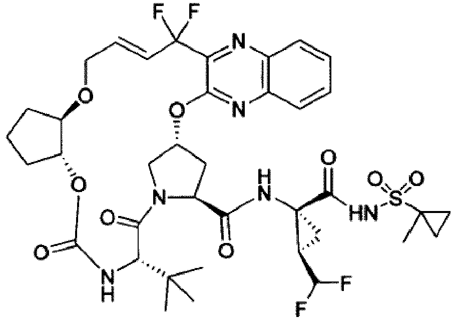 and wherein Compound 2 is 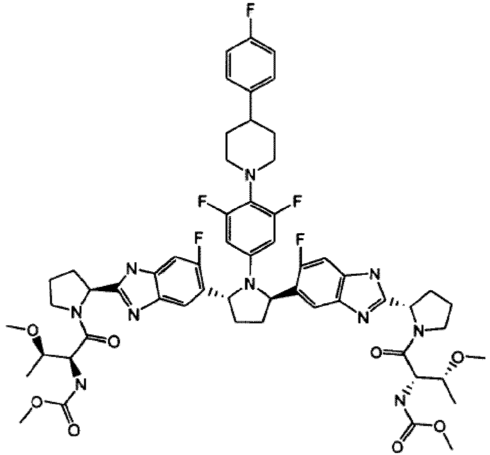 The treatments comprise administering at least two direct acting antiviral agents (DAAs) and ribavirin to the subject for a duration lasting 12 weeks or 16 weeks. The at least two DAAs comprise Compound 1 (or a pharmaceutically acceptable salt thereof) and Compound 2 (or a pharmaceutically acceptable salt thereof). Preferably, the duration of the treatment is 12 weeks. Preferably, the two or more DAAs are administered in amounts effective to provide a sustained virological response (SVR) or achieve another desired measure of effectiveness in the subject. The subject is not administered interferon during the treatment regimen. Put another way, the treatments exclude the administration of interferon to the subject, thereby avoiding the side effects associated with interferon.

Another aspect of the present invention features combinations for treating a population of subjects having HCV infection. The treatments comprise administering at least two DAAs and ribavirin to the subjects for a duration of no more than 12 weeks. The at least two DAAs comprise Compound 1 (or a pharmaceutically acceptable salt thereof) and Compound 2 (or a pharmaceutically acceptable salt thereof). Preferably, the at least two DAAs are administered to the subjects in amounts effective to result in SVR or another measure of effectiveness in at least about 70% of the population, preferably at least about 80% of the population, or more preferably at least about 90% of the population.

In any treatment described herein, the at least two DAAs comprise (a) Compound 1 or a pharmaceutically acceptable salt thereof, and (b) Compound 2 or a pharmaceutically acceptable salt thereof and (c) an HCV polymerase inhibitor or pharmaceutically acceptable salt thereof. The at least two DAAs can also optionally comprise another anti-HCV agent. The other optional anti-HCV agent can be selected from protease inhibitors, nucleoside or nucleotide polymerase inhibitors, non-nucleoside polymerase inhibitors, NS3B inhibitors, NS4A inhibitors, NS5A inhibitors, NS5B inhibitors, cyclophilin inhibitors, or combinations thereof. For another example, the DAAs used in a method of the present invention may comprise or consist of (a) Compound 1 or a pharmaceutically acceptable salt thereof, (b) Compound 2 or a pharmaceutically acceptable salt thereof, and (c) a HCV polymerase inhibitor, wherein said HCV polymerase inhibitor can be a nucleotide or nucleoside polymerase inhibitor or a non-nucleoside or non-nucleotide polymerase inhibitor.

Examples of the other optional antic-HCV agent include PSI-7977 (sofosbuvir), PSI-938, BMS-790052 (daclatasvir), BMS-650032 (asunaprevir), BMS-791325, GS-5885 (ledipasvir), GS-9451 (tegobuvir), GS-9190, GS-9256, BI-201335, BI-27127, telaprevir, VX-222, TMC-435 (simepravir), MK-5172, MK-7009 (vaniprevir), danoprevir, R7128 (mericitabine), and any combination thereof.

In any treatment described herein, the DAAs can be administered in any effective dosing schemes and/or frequencies; for example, they can each be administered daily. Each DAA can be administered either separately or in combination, and each DAA can be administered once a day, twice a day, or three times a day. Preferably, Compound 1 (or a pharmaceutically acceptable salt thereof) and Compound 2 (or a pharmaceutically acceptable salt thereof) are administered once daily.

Preferably, Compound 1 (or a pharmaceutically acceptable salt thereof) is administered from 100 mg to 600 mg once daily, and Compound 2 (or a pharmaceutically acceptable salt thereof) is administered from 50 to 500 mg once daily. More preferably, Compound 1 (or a pharmaceutically acceptable salt thereof) is administered from 200 mg to 600 mg once daily, and Compound 2 (or a pharmaceutically acceptable salt thereof) is administered from 100 to 500 mg once daily. Highly preferably, Compound 1 (or a pharmaceutically acceptable salt thereof) is administered from 400 mg to 600 mg once daily, and Compound 2 (or a pharmaceutically acceptable salt thereof) is administered from 100 to 500 mg once daily. For instance, Compound 1 (or a pharmaceutically acceptable salt thereof) can be administered 400 mg once daily, and Compound 2 (or a pharmaceutically acceptable salt thereof) is administered 120 mg once daily. For another instance, Compound 1 (or a pharmaceutically acceptable salt thereof) can be administered 400 mg once daily, and Compound 2 (or a pharmaceutically acceptable salt thereof) can be administered 240 mg once daily.

In yet another aspect, the present invention features a combination of Compound 1 (or a pharmaceutically acceptable salt thereof) and Compound 2 (or a pharmaceutically acceptable salt thereof), together with ribavirin, for use to treat HCV infection as set forth in the claims. The treatment comprises administering the DAAs and ribavirin to a subject infected with HCV The duration of the treatment regimen may be twelve weeks. The treatment does not include administering interferon. Compound 1 (or the salt thereof) and Compound 2 (or the salt thereof) can be administered concurrently or sequentially. Preferably, Compound 1 (or the salt thereof) and Compound 2 (or the salt thereof) can be administered once daily. As an example, the patient being treated is infected with HCV genotype 1, such as genotype 1a or 1b. As another example, the patient is infected with HCV genotype 2. As another example, the patient is infected with HCV genotype 3. As another example, the patient is infected with HCV genotype 4. As another example, the patient is infected with HCV genotype 5. As another example, the patient is infected with HCV genotype 6. As yet another example, the patient is a HCV-treatment naive patient, a HCV-treatment experienced patient, an interferon non-responder (e.g., a null responder), or not a candidate for interferon treatment. As used in this application, the interferon non-responder patients include partial interferon responders and interferon rebound patients. *See* GUIDANCE FOR INDUSTRY - CHRONIC HEPATITIS C VIRUS INFECTION: DEVELOPING DIRECT-ACTING ANTIVIRAL AGENTS FOR TREATMENT (FDA, September 2010, draft guidance) for the definitions of naive, partial responder, responder relapser (i.e., rebound), and null responder patients. The interferon non-responder patients also include null responder patients. In one example of this aspect of the invention, the treatment lasts for 12 weeks, and the subject being treated is a naive patient infected with HCV genotype 1. In yet another example, the treatment lasts for 12 weeks, and the subject being treated is a naive patient infected with HCV genotype 3.In yet another example, the treatment lasts for 12 weeks, and the subject being treated is a non-responder (e.g., a null responder) infected with HCV genotype 1. In yet another example, the treatment lasts for 12 weeks, and the subject being treated is a non-responder (e.g., a null responder) infected with HCV genotype 3.

A treatment regimen of the present invention generally constitutes a complete treatment regimen, i.e., no subsequent interferon-containing regimen is intended. Thus, a treatment or use described herein generally does not include any subsequent interferon-containing treatment.

The present application is defined in the appended claims. Subject matters which are not encompassed by the scope of the claims do not form part of the present invention.

### BRIEF DESCRIPTION OF THE DRAWINGS

Figure 1 shows the predicted median SVR percentages and 90% SVR confidence intervals for interferon-free, 2-DAA regimens comprising the use of Compound 1 (400 mg once daily) and Compound 2 (120 mg once daily) to treat genotype 1 naive subjects.
Figure 2 illustrates the predicted median SVR percentages and 90% SVR confidence intervals for interferon-free, 2-DAA regimens comprising the use of Compound 1 (400 mg once daily) and Compound 2 (60 mg once daily) to treat genotype 1 naive subjects.
Figure 3 depicts the predicted median SVR percentages and 90% SVR confidence intervals for interferon-free, 2-DAA regimens comprising the use of Compound 1 (600 mg once daily) and Compound 2 (480 mg once daily) to treat genotype 1 naive subjects.
Figure 4 shows the predicted median SVR percentages and 90% SVR confidence intervals for interferon-free, 2-DAA regimens comprising the use of Compound 1 (400 mg once daily) and Compound 2 (120 mg once daily) to treat genotype 3 naive subjects.
Figure 5 illustrates the predicted median SVR percentages and 90% SVR confidence intervals for interferon-free, 2-DAA regimens comprising the use of Compound 1 (400 mg once daily) and Compound 2 (60 mg once daily) to treat genotype 3 naive subjects.
Figure 6 shows the predicted median SVR percentages and 90% SVR confidence intervals for interferon-free, 2-DAA regimens comprising the use of Compound 1 (600 mg once daily) and Compound 2 (480 mg once daily) to treat genotype 3 naive subjects.
Figure 7 depict the synergistic effect of the combination of Compound 1 and Compound 2 on HCV inhibition *in vitro.*

### DETAILED DESCRIPTION OF THE INVENTION

The treatments described herein include administering Compound 1 (or a pharmaceutically acceptable salt thereof) and Compound 2 (or a pharmaceutically acceptable salt thereof) and ribavirin to a subject in need thereof. Compound 1 has the following structure: 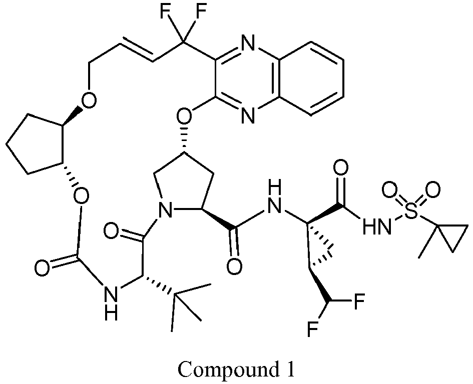 Compound 1 is a potent HCV protease inhibitor and is described in U.S. Patent Application Publication No. 2012/0070416.

Compound 2 has the following structure: 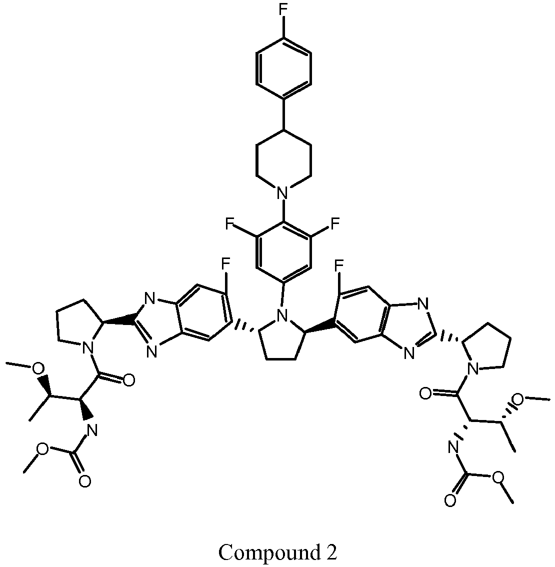 Compound 2 is a potent NS5A inhibitor and is described in U.S. Patent Application Publication No. 2012/0220562.

The current standard of care (SOC) for the treatment of HCV includes a course of treatment of interferon, e.g. pegylated interferon (e.g., pegylated interferon-alpha-2a or pegylated interferon-alpha-2b, such as PEGASYS by Roche, or PEG-INTRON by Schering-Plough) and the antiviral drug ribavirin (e.g., COPEGUS by Roche, REBETOL by Schering-Plough, or RIBASPHERE by Three Rivers Pharmaceuticals). The treatment often lasts for 24-48 weeks, depending on hepatitis C virus genotype. Other interferons include interferon-alpha-2a (e.g., Roferon-A by Roche), interferon-alpha-2b (e.g., Intron-A by Schering-Plough), and interferon alfacon-1 (consensus interferon) (e.g., Infergen by Valeant).

The interferon-based treatment may be physically demanding, and can lead to temporary disability in some cases. A substantial proportion of patients will experience a panoply of side effects ranging from a "flu-like" syndrome (the most common, experienced for a few days after the weekly injection of interferon) to severe adverse events including anemia, cardiovascular events and psychiatric problems such as suicide or suicidal ideation. The latter are exacerbated by the general physiological stress experienced by the patients.

The treatments described herein provide effective treatment of HCV infection without the use of interferon and for a shorter period of time, for example, a treatment duration of twelve weeks, or sixteen weeks.

The treatments described herein comprise administering at least two DAAs and ribavirin, in the absence of interferon, to the subject for a duration of twelve weeks, or sixteen weeks. Put another way, the treatments exclude interferon. The at least two DAAs comprise Compound 1 (or a pharmaceutically acceptable salt thereof) and Compound 2 (or a pharmaceutically acceptable salt thereof), which can be co-administered, or administered separately or independently, with the same or different dosing frequencies. Preferably, the at least two DAAs are administered once a day. They can also be administered, for example, twice a day or three times a day.

Various measures may be used to express the effectiveness of a treatment of the present invention. One such measure is SVR, which, as used herein, means that the virus is undetectable at the end of therapy and for at least 8 weeks after the end of therapy (SVR8); preferably, the virus is undetectable at the end of therapy and for at least 12 weeks after the end of therapy (SVR12); more preferably, the virus is undetectable at the end of therapy and for at least 16 weeks after the end of therapy (SVR16); and highly preferably, the virus is undetectable at the end of therapy and for at least 24 weeks after the end of therapy (SVR24). SVR24 is often considered as a functional definition of cure; and a high rate of SVR at less than 24 week post-treatment (e.g., SVR8 or SVR12) can be predictive of a high rate of SVR24.

In some embodiments, a treatment regimen according to the invention comprises treating a population of subjects having HCV infection (e.g. treatment naive subjects), and the regimen comprises administering at least two DAAs and ribavirin to the subjects for a duration of 12 weeks, or 16 weeks, wherein the at least two DAAs comprise Compound 1 (or a pharmaceutically acceptable salt thereof) and Compound 2 (or a pharmaceutically acceptable salt thereof), and are administered to the subjects in amounts effective to provide an SVR (e.g., SVR12 or SVR24) in at least about 70% of the population, alternatively at least about 75% of the population, alternatively at least about 80% of the population, alternatively at least about 85% of the population, alternatively at least about 90% of the population, alternatively at least about 95% of the population, alternatively about 100% of the population. In some embodiments, a treatment regimen of the invention comprises treating a population of IFN experienced subjects (e.g., interferon non-responders) having HCV infection, and the method comprises administering at least two DAAs and ribavirin to the subjects for a duration of 12 weeks, or 16 weeks, wherein the at least two DAAs comprise Compound 1 (or a pharmaceutically acceptable salt thereof) and Compound 2 (or a pharmaceutically acceptable salt thereof), and are administered to the subjects in amounts effective to provide an SVR (e.g., SVR12 or SVR24) in at least about 50% of the population, alternatively at least about 55% of the population, alternatively at least about 60% of the population, alternatively at least about 65% of the population, alternatively at least about 70% of the population, alternatively at least about 75% of the population, alternatively at least about 80% of the population, alternatively at least about 85% of the population, alternatively at least about 90% of the population, alternatively at least about 95% of the population, or alternatively about 100% of the population.

It was unexpected that an interferon-free treatment using a combination of Compound 1 (or a pharmaceutically acceptable salt thereof) and Compound 2 (or a pharmaceutically acceptable salt thereof) and ribavirin, and for a duration of no more than 12 weeks, can achieve significant SVR.

In yet another aspect, the present invention features combination for use in a method of treating HCV infection, the treatment comprising administering to a patient in need thereof ribavirin and an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 16 weeks and does not include administration of any interferon. The DAAs can be administered at the same or different dosing frequencies. The patient being treated can be a treatment naive patient; a treatment experienced patient, including, a relapser, an interferon partial responder, an interferon non-responder, or a null responder; or a patient unable to take interferon. The patient may be infected with, for example, HCV genotype 1, such as HCV genotype 1a or HCV genotype lb; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs, including HCV protease inhibitors, HCV polymerase inhibitors, or HCV NS5A inhibitors. Examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a combination for use in treating HCV infection, the treatment comprising administering to a patient in need thereof ribavirin and an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 12 weeks and does not include administration of any interferon. The DAAs can be administered at the same or different dosing frequencies. The patient being treated can be a treatment naive patient; a treatment experienced patient, including a relapser, an interferon partial responder, an interferon non-responder, or a null responder; or a patient unable to take interferon. The patient may be infected with, for example, HCV genotype 1, such as HCV genotype 1a or HCV genotype lb; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs, including HCV protease inhibitors, HCV polymerase inhibitors, or HCV NS5A inhibitors. Examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir. As used in this application, a HCV polymerase inhibitor can be a nucleoside polymerase inhibitor, a nucleotide polymerase inhibitor, a non-nucleoside polymerase inhibitor, or a non-nucleotide polymerase inhibitor.

In each aspect, embodiment, example or treatment described herein, Compound 1 (or a pharmaceutically acceptable salt thereof) may be administered from 100 mg to 600 mg once daily, and Compound 2 (or a pharmaceutically acceptable salt thereof) may be administered from 50 to 500 mg once daily. More preferably, Compound 1 (or a pharmaceutically acceptable salt thereof) is administered from 200 mg to 600 mg once daily, and Compound 2 (or a pharmaceutically acceptable salt thereof) is administered from 100 to 500 mg once daily. Highly preferably, Compound 1 (or a pharmaceutically acceptable salt thereof) is administered from 400 mg to 600 mg once daily, and Compound 2 (or a pharmaceutically acceptable salt thereof) is administered from 100 to 500 mg once daily. Preferably, Compound 1 (or a pharmaceutically acceptable salt thereof) can be administered 400 mg once daily, and Compound 2 (or a pharmaceutically acceptable salt thereof) is administered 120 mg once daily. Also preferably, Compound 1 (or a pharmaceutically acceptable salt thereof) can be administered 400 mg once daily, and Compound 2 (or a pharmaceutically acceptable salt thereof) can be administered 240 mg once daily.

In each aspect, embodiment, example or treatment described herein, ribavirin can be any suitable form or formulation of ribavirin including its well-known pro-drugs. Exemplary formulations of ribavirin include COPEGUS®, REBETOL® and RIBASPHERE®. An exemplary pro-drug of ribavirin is taribavirin having the chemical name of 1-β-D-ribofuranosyl-1,2,4-triazole-3-carboxamidine. Ribavirin and taribavirin may be administered in accordance with ribavirin and taribavirin administration well known in the art. In some embodiments, COPEGUS® or REBETOL® is administered in a daily dosage amount of from about 500 mg to about 1500 mg in one dose or in divided doses. In some embodiments, COPEGUS® or REBETOL® is administered in a daily dosage amount of about 800 mg. In some embodiments, REBETOL® is administered in a daily dosage amount of about 1000 mg. In some embodiments, COPEGUS® or REBETOL® is administered in a daily dosage amount of about 1200 mg. In some embodiments, REBETOL® is administered in a daily dosage amount of about 1400 mg. Suitable dosages of ribavirin are often dependent on the weight of the subject, for example about 1000-1200 mg. Suitable total daily dosages of ribavirin include about 400 mg to about 1400 mg a day, alternatively about 800 mg to about 1400 mg per day, alternatively about 400 mg to about 1200 mg, alternatively about 800 mg to about 1200 mg.

A combination of the present invention can be used to treat a naive patient or a treatment experienced patient. Treatment experienced patients include interferon non-responders (e.g., null responders), partial responders, and relapsers. A combination of the present invention can also be used to treat patients who are not candidates for interferon treatment. Patients who are not candidates for interferon treatment include one or more of the following groups: patients intolerant to interferon, patients who refuse to take interferon treatment, patients with medical conditions which preclude them from taking interferon, and patients who have an increased risk of side effects or infection by taking interferon.

In any treatment described herein, one or more additional DAAs can be optionally used in the treatment regimen in addition to Compound 1 (or a salt thereof) and Compound 2 (or a salt thereof) and the HCV polymerase inhibitor or salt thereof specified in the claims. These additional DAAs can be HCV protease inhibitors, HCV nucleoside or nucleotide polymerase inhibitors, HCV non-nucleoside polymerase inhibitors, HCV NS3B inhibitors, HCV NS4A inhibitors, HCV NS5A inhibitors, HCV NS5B inhibitors, HCV entry inhibitors, cyclophilin inhibitors, or combinations thereof.

Preferred HCV protease inhibitors for this purpose include telaprevir (Vertex), boceprevir (Merck), BI-201335 (Boehringer Ingelheim), GS-9451 (Gilead), and BMS-650032 (BMS). Other suitable protease inhibitors include ACH-1095 (Achillion), ACH-1625 (Achillion), ACH-2684 (Achillion), AVL-181 (Avila), AVL-192 (Avila), BMS-650032 (BMS), danoprevir (RG7227/ITMN-191, Roche), GS-9132 (Gilead), GS-9256 (Gilead), IDX-136 (Idenix), IDX-316 (Idenix), IDX-320 (Idenix), MK-5172 (Merck), narlaprevir (Schering-Plough Corp), PHX-1766 (Phenomix), TMC-435 (Tibotec), vaniprevir (MK-7009, Merck), VBY708 (Virobay), VX-500 (Vertex), VX-813 (Vertex), VX-985 (Vertex), or a combination thereof.

Preferred non-nucleoside HCV polymerase inhibitors for use in the present invention include GS-9190 (Gilead), BI-207127 (Boehringer Ingelheim), and VX-222 (VCH-222) (Vertex & ViraChem). Preferred nucleotide HCV polymerase inhibitors include PSI-7977 (Gilead), and PSI-938 (Gilead). Other suitable and non-limiting examples of suitable HCV polymerase inhibitors include ANA-598 (Anadys), BI-207127 (Boehringer Ingelheim), BILB-1941 (Boehringer Ingelheim), BMS-791325 (BMS), filibuvir, GL59728 (Glaxo), GL60667 (Glaxo), GS-9669 (Gilead), IDX-375 (Idenix), MK-3281 (Merck), tegobuvir, TMC-647055 (Tibotec), VCH-759 (Vertex & ViraChem), VCH-916 (ViraChem), VX-759 (Vertex), GS-6620 (Gilead), IDX-102 (Idenix), IDX-184 (Idenix), INX-189 (Inhibitex), MK-0608 (Merck), RG7128 (Roche), TMC64912 (Medivir), GSK625433 (GlaxoSmithKline), BCX-4678 (BioCryst), ALS-2200 (Alios BioPharma/Vertex), ALS-2158 (Alios BioPharma/Vertex), or a combination thereof. A polymerase inhibitor may be a nucleoside or nucleotide polymerase inhibitor, such as GS-6620 (Gilead), IDX-102 (Idenix), IDX-184 (Idenix), INX-189 (Inhibitex), MK-0608 (Merck), PSI-7977 (Gilead), PSI-938 (Gilead), RG7128 (Roche), TMC64912 (Medivir), ALS-2200 (Alios BioPharma/Vertex), ALS-2158 (Alios BioPharma/Vertex), or a combination therefore. A polymerase inhibitor may also be a non-nucleoside polymerase inhibitor, such as PF-00868554 (Pfizer), ANA-598 (Anadys), BI-207127 (Boehringer Ingelheim), BILB-1941 (Boehringer Ingelheim), BMS-791325 (BMS), filibuvir, GL59728 (Glaxo), GL60667 (Glaxo), GS-9669 (Gilead), IDX-375 (Idenix), MK-3281 (Merck), tegobuvir (Gilead),, TMC-647055 (Tibotec), VCH-759 (Vertex & ViraChem), VCH-916 (ViraChem), VX-222 (VCH-222) (Vertex & ViraChem), VX-759 (Vertex), or a combination thereof.

Preferred NS5A inhibitors include BMS-790052 (BMS) and GS-5885 (Gilead). Examples of suitable NS5A inhibitors include GSK62336805 (GlaxoSmithKline), ACH-2928 (Achillion), AZD2836 (Astra-Zeneca), AZD7295 (Astra-Zeneca), BMS-790052 (BMS), BMS-824393 (BMS), GS-5885 (Gilead), PPI-1301 (Presidio), PPI-461 (Presidio) A-831 (Arrow Therapeutics), A-689 (Arrow Therapeutics) or a combination thereof.

Examples of suitable cyclophilin inhibitors include alisporovir (Novartis & Debiopharm), NM-811 (Novartis), SCY-635 (Scynexis), or a combination thereof.

Examples of suitable HCV entry inhibitors include ITX-4520 (iTherx), ITX-5061 (iTherx), or a combination thereof.

Specific examples of other DAA agents that are suitable for inclusion in a method of the present invention include AP-H005, A-831 (Arrow Therapeutics) (NS5A inhibitor), A-689 (Arrow Therapeutics) (NS5A inhibitor), INX08189 (Inhibitex) (polymerase inhibitor), ITMN-191 (Intermune/Roche) (NS3/4A Protease inhibitor), VBY-376 (Protease Inhibitor) (Virobay), ACH-1625 (Achillion, Protease inhibitor), IDX136 (Idenix, Protease Inhibitor), IDX316 (Idenix, Protease inhibitor), VX-813 (Vertex), SCH 900518 (Schering-Plough), TMC-435 (Tibotec), ITMN-191 (Intermune, Roche), MK-7009 (Merck), IDX-PI (Novartis), R7128 (Roche), PF-868554 (Pfizer) (non-nucleoside polymerase inhibitor), PF-4878691 (Pfizer), IDX-184 (Idenix), IDX-375 (Idenix, NS5B polymerase inhibitor), PPI-461 (Presidio), BILB-1941 (Boehringer Ingelheim), GS-9190 (Gilead), BMS-790052 (BMS), CTS-1027 (Conatus), GS-9620 (Gilead), PF-4878691 (Pfizer), RO5303253 (Roche), ALS-2200 (Alios BioPharma/Vertex), ALS-2158 (Alios BioPharma/Vertex), GSK62336805 (GlaxoSmithKline), or any combinations thereof.

The chemical structures of some of these optional HCV inhibitors are provided below:

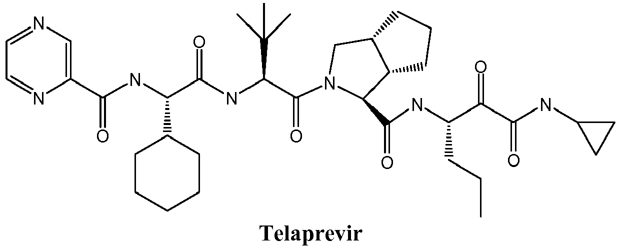

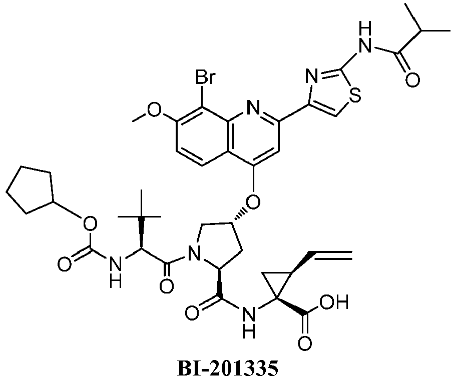

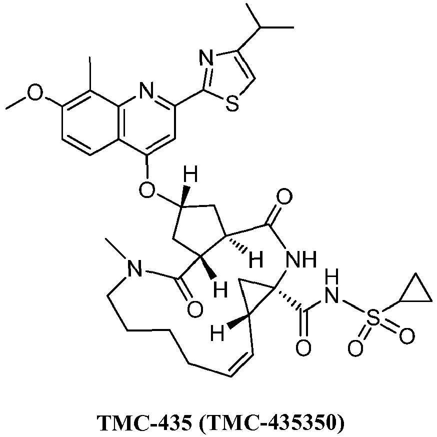

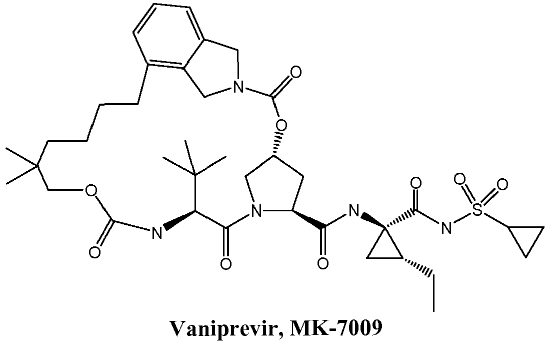

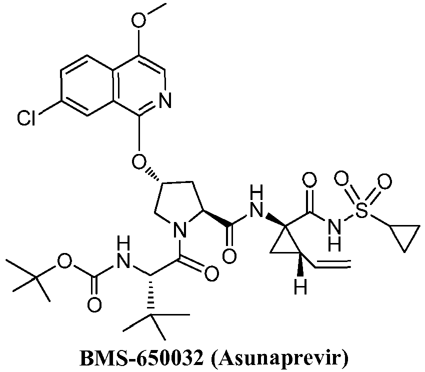

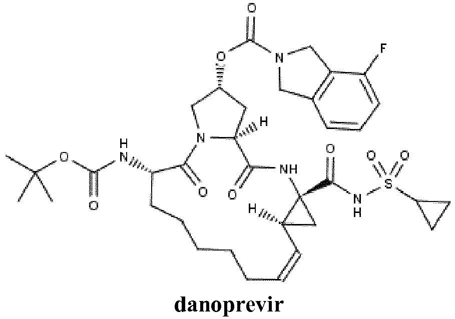

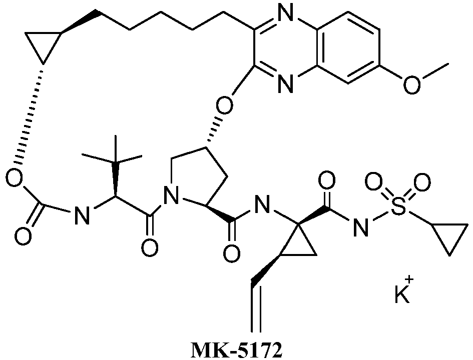

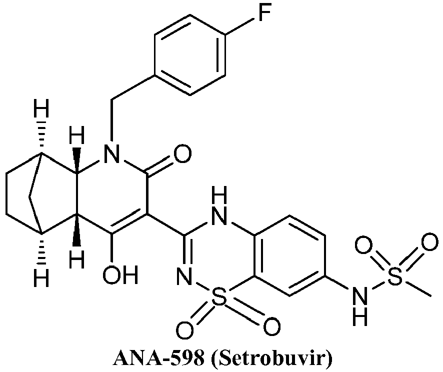

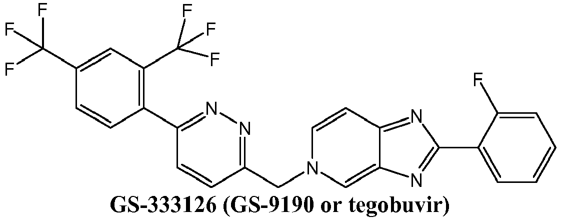

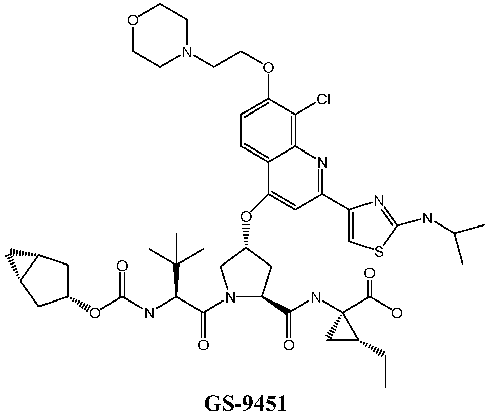

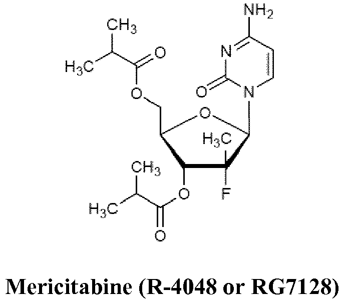

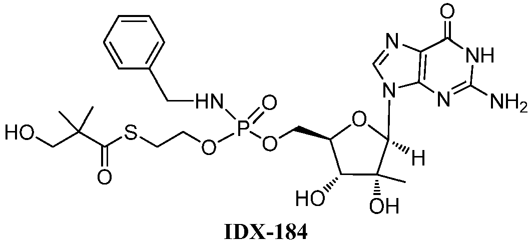

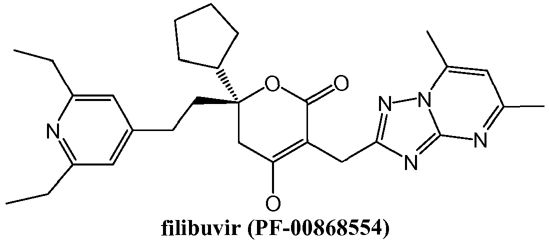

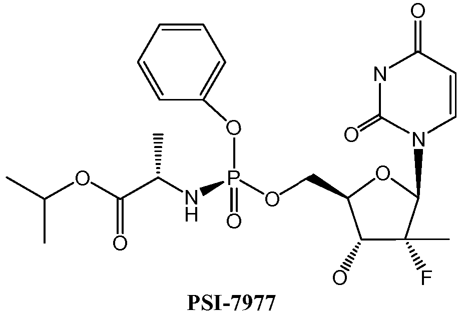

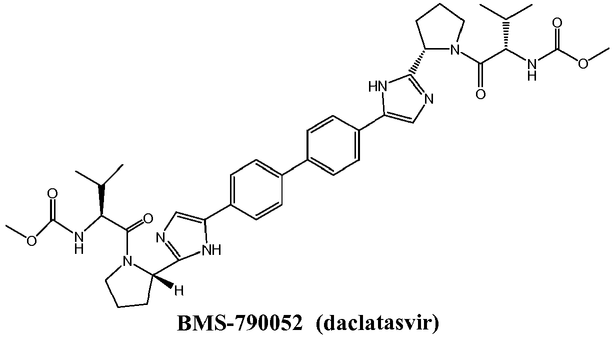

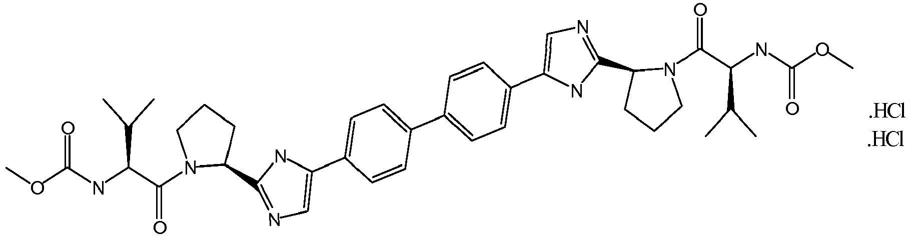

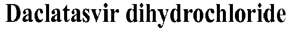

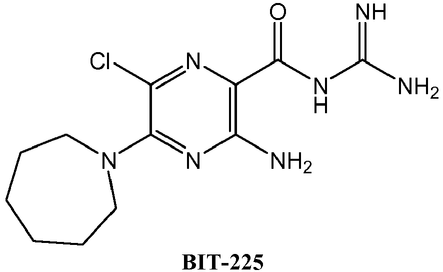

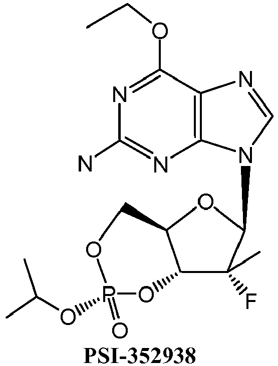

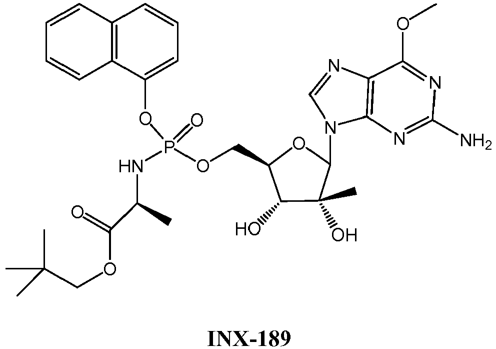

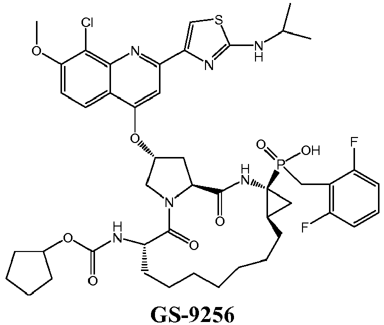

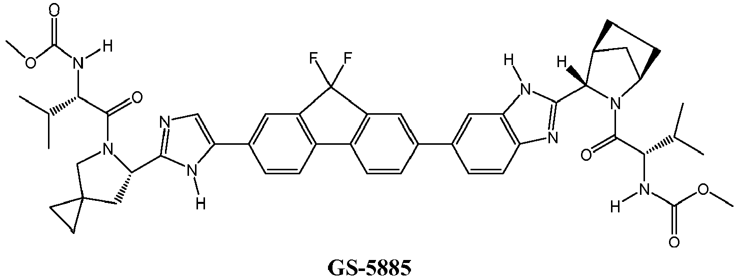

Any HCV inhibitor or DAA described herein encompasses its suitable salt forms when it is used in therapeutic treatments or pharmaceutical formulations.

In some embodiments, the present invention features combinations for treating patients infected with HCV genotype 1, such as 1a or 1b. The treatments comprise administering to such a patient a combination of at least 2 DAAs and ribavirin for 12 weeks (the duration being 12 weeks), wherein the treatment does not include administration of interferon, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment.

In some embodiments, the present invention features combinations for use in treating patients with HCV genotype 2 or 3 infection. The treatments comprise administering to such a patient a combination of at least 2 DAAs and ribavirin for 12 weeks (the duration being 12 weeks), wherein the treatment does not include administration of interferon, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients.

In some embodiments, the present invention features combinations for treating patients with HCV genotype 2 infection. The treatments comprise administering to such a patient a combination of at least 2 DAAs and ribavirin for 12 weeks (the duration being 12 weeks), wherein the treatment does not include administration of interferon, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients.

In some embodiments, the present invention features combinations for treating patients with HCV genotype 3 infection. The treatments comprise administering to such a patient a combination of at least 2 DAAs and ribavirin for 12 weeks (e.g., the duration being 12 weeks), wherein the treatment does not include administration of interferon, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients.

In some embodiments, the present invention features combinations for treating patients with HCV genotype 4 infection. The treatments comprise administering to such a patient a combination of at least 2 DAAs and ribavirin for 12 weeks (the duration being 12 weeks), wherein the treatment does not include administration of interferon, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients.

In some embodiments, the present invention features combinations for treating patients with HCV genotype 5 infection. The treatments comprise administering to such a patient a combination of at least 2 DAAs and ribavirin for 12 weeks (the duration being 12 weeks), wherein the treatment does not include administration of interferon, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients.

In some embodiments, the present invention features combinations for treating patients with HCV genotype 6 infection. The treatments comprise administering to such a patient a combination of at least 2 DAAs and ribavirin for 12 weeks (the duration being 12 weeks), wherein the treatment does not include administration of interferon, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients.

It will be understood that the specific dose level for any particular patient will depend upon a variety of factors including the activity of the specific compound employed, the age, body weight, general health, sex, diet, time of administration, route of administration, rate of excretion, drug combination, and the severity of the disease undergoing therapy.

In any treatment described herein, Compound 1 (or a pharmaceutically acceptable salt thereof) and Compound 2 (a pharmaceutically acceptable salt thereof) may be co-formulated in a single dosage form. Non-limiting examples of suitable dosage forms include liquid or solid dosage forms. Preferably, Compound 1 and Compound 2 are formulated in a single solid dosage form in which at least one of the DAAs is in an amorphous form, or highly preferably molecularly dispersed, in a matrix which comprises a pharmaceutically acceptable water-soluble polymer and a pharmaceutically acceptable surfactant. The other DAAs can also be in an amorphous form or molecularly dispersed in the matrix, or formulated in different form(s) (e.g., in a crystalline form). More preferably, each of the two DAAs is in an amorphous form, or highly preferably molecularly dispersed, in a matrix which comprises a pharmaceutically acceptable water-soluble polymer and a pharmaceutically acceptable surfactant.

In any treatment described herein, the patient being treated can be a treatment-naive patient.

In any treatment described herein, the patient being treated can be an interferon non-responder.

In any method treatment herein, the patient being treated can be an interferon null-responder.

In any treatment described herein, the patient being treated can be without cirrhosis.

In any treatment described herein, the patient being treated can be a cirrhotic patient.

In any treatment described herein, the patient being treated can be a patient with compensated cirrhosis.

### Example 1. Clinical Modeling for Interferon-free DAA Combination Therapies

Treatment regimens comprising administration of Compound 1 and Compound 2 were evaluated using clinical models described in U.S. Patent Application Publication No. 2013/0102526, filed October 19, 2012 and entitled "Methods for Treating HCV. These treatment regimens comprised administration of Compound 1 and Compound 2, but did not include administration of either interferon or ribavirin. However, similar SVR rates are expected when ribavirin is added to these regimens.

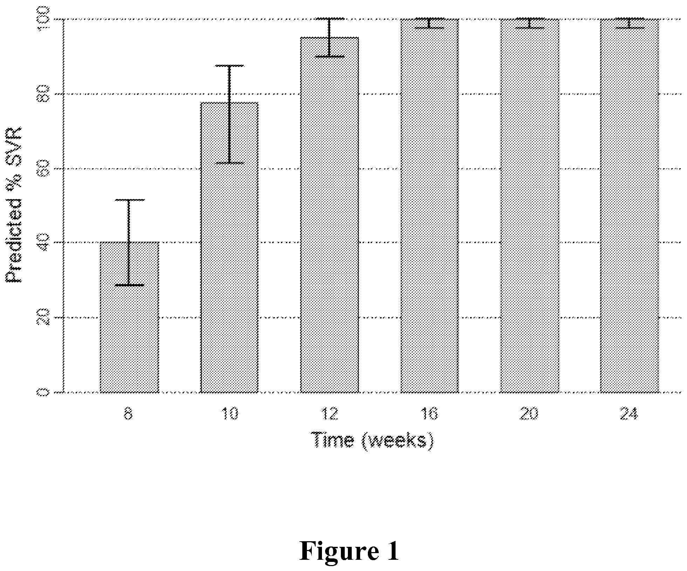

Figure 1 shows the predicted median SVR percentages and 90% SVR confidence intervals for 2-DAA regimens consisting of the use of Compound 1 (400 mg once daily) and Compound 2 (120 mg once daily) to treat genotype 1 naive subjects. Different treatment durations were assessed. The predicted SVR rate for a 12-week treatment was about 95%. As used in all of the figures of the present application, the vertical bar at the top of each SVR percentage column represents the 90% SVR confidence interval, and the x-axis ("Time (weeks)") indicates the duration of each treatment regimen.

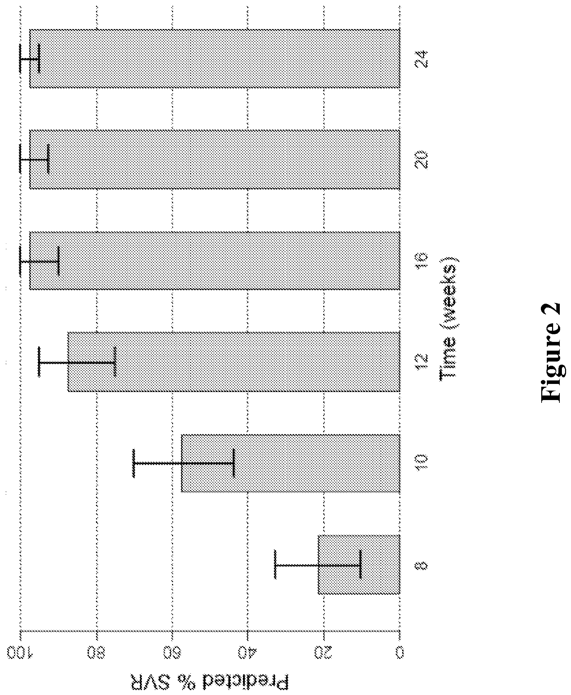

Figure 2 illustrates the predicted median SVR percentages and 90% SVR confidence intervals for 2-DAA regimens consisting of the use of Compound 1 (400 mg once daily) and Compound 2 (60 mg once daily) to treat genotype 1 naive subjects. Different treatment durations were assessed. The predicted SVR rate for a 12-week treatment was about 85-90%.

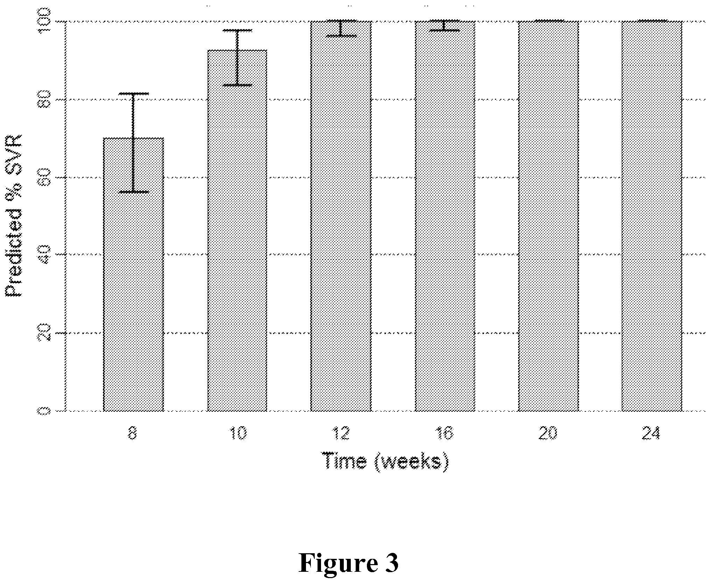

Figure 3 shows the predicted median SVR percentages and 90% SVR confidence intervals for 2-DAA regimens consisting of the use of Compound 1 (600 mg once daily) and Compound 2 (480 mg once daily) to treat genotype 1 naive subjects. Different treatment durations were assessed. The predicted SVR rate for a 12-week treatment was about 100%.

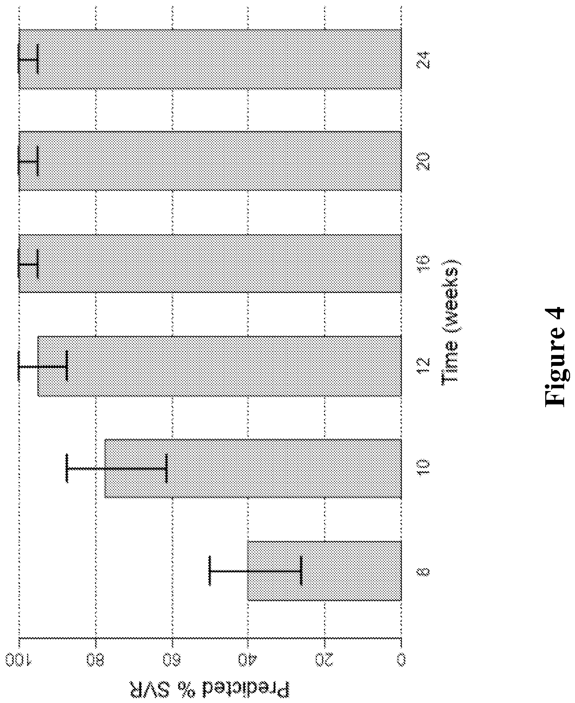

Figure 4 depicts the predicted median SVR percentages and 90% SVR confidence intervals for 2-DAA regimen consisting of the use of Compound 1 (400 mg once daily) and Compound 2 (120 mg once daily) to treat genotype 3 naive subjects. Different treatment durations were assessed. The predicted SVR rate for a 12-week treatment was about 95%.

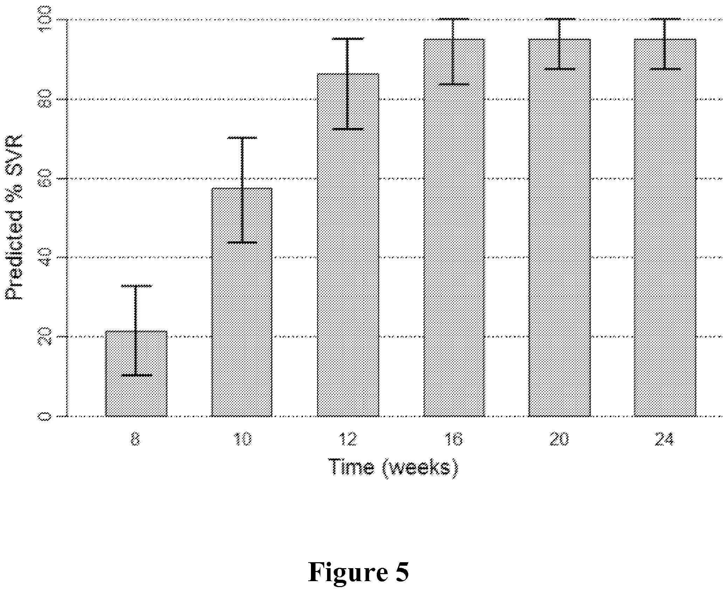

Figure 5 illustrates the predicted median SVR percentages and 90% SVR confidence intervals for 2-DAA regimen consisting of the use of Compound 1 (400 mg once daily) and Compound 2 (60 mg once daily) to treat genotype 3 naive subjects. Different treatment durations were assessed. The predicted SVR rate of a 12-week treatment was about 85-90%.

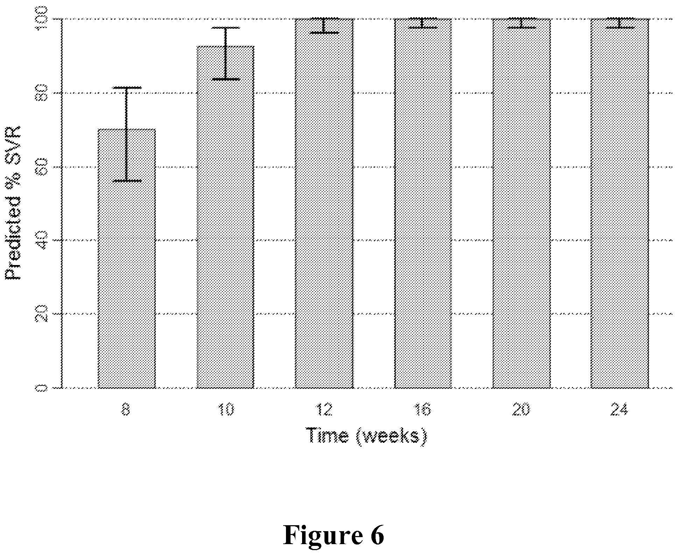

Figure 6 shows the predicted median SVR percentages and 90% SVR confidence intervals for 2-DAA regimens consisting of the use of Compound 1 (600 mg once daily) and Compound 2 (480 mg once daily) to treat genotype 3 naive subjects. Different treatment durations were assessed. The predicted SVR rate of a 12-week treatment was about 100%.

### Example 2. Combination of Compound 1 and Compound 2 In Vitro

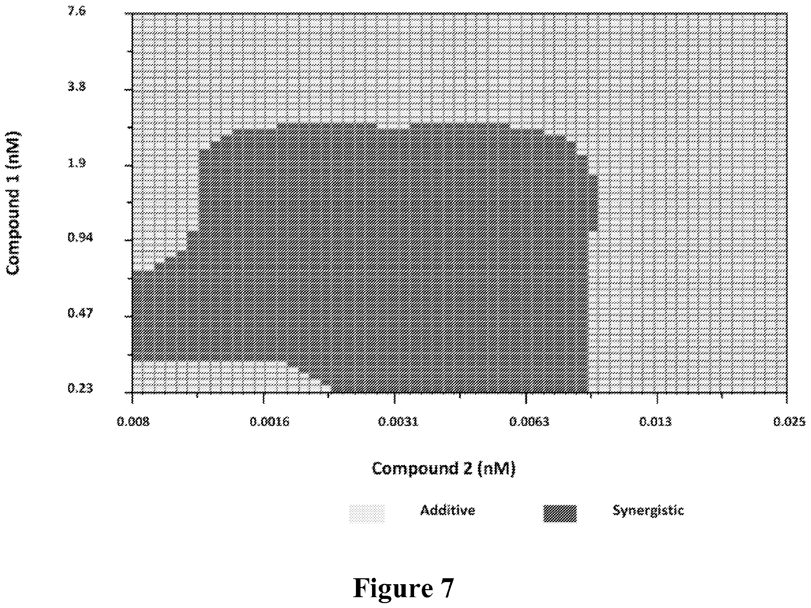

Figure 7 shows that the combination of Compound 1 and Compound 2 exhibits significant synergistic effect on HCV inhibition as tested in HCV GT 1b Con-1 replication cells. The result was generated using Prichard and Shipman model (Prichard et al. ANTIVIRAL RESEARCH 14:181-205 (1990)).

Compound 1 inhibited replication of HCV stable subgenomic replicons containing NS3 genes from GT 1a, 1b, 2a, 3a, 4a, or 6a with EC₅₀ values ranging from 0.85 to 2.8 nM. Of note, Compound 1 was potent against replicon containing GT3a protease, with an EC₅₀ value of 1.6 nM. Compound 1 retained its activity against common GTla and 1b variants at NS3 amino acid positions 155 and 168 that conferred resistance to other HCV protease inhibitors (Pis). Resistant colony selection studies in GTla and 1b subgenomic replicon cells identified A156T in GTla and A156V in GT1b as the most frequent variants, which conferred 1400- and 1800-fold reduced susceptibility to Compound 1, respectively. However, these variants had *in vitro* replication capacities of only 1.5% and 9.2% that of their corresponding wild-type replicons. In a replicon containing GT3a NS3 protease, Compound 1 selected very few colonies at concentrations ≥ 100-fold over its EC50 value. The colonies that survived the selection contained either A156G alone, or Q168R co-selected with Y56H, which conferred 1500- or 1100-fold loss in susceptibility to Compound 1, respectively.

**Table 2. Antiviral Activity of Compound 1 in the HCV Subgenomic Stable Replicon Cell Culture Assay**

| | | **0% Human Plasma^{a}** |
|---|---|---|
| **HCV Replicon Subtype** | N^{b} | **Mean EC₅₀, nM, ± Std. Dev.** |
| Genotype 1a | 9 | 0.85 ± 0.15 |
| Genotype 1b | 8 | 0.94 ± 0.35 |
| Genotype 2a | 2 | 2.7 ± 1.1 |
| Genotype 3a | 2 | 1.6 ± 0.49 |
| Genotype 4a | 4 | 2.8 ± 0.41 |
| Genotype 6a | 4 | 0.86 ± 0.11 |

| | | |
|---|---|---|
| a. The 0% human plasma assay contains 5% fetal bovine serum b. Number of independent replicates | | |

**Table 3. Antiviral Activity of Compound 1 in the HCV Subgenomic Stable Replicon Cell Culture Assay**

| | | **40% Human Plasma^{a}** |
|---|---|---|
| **HCV Replicon Subtype** | **N^{b}** | **Mean EC₅₀, nM, ± Std. Dev.** |
| Genotype 1a | 10 | 5.3 ± 1.0 |
| Genotype 1b | 8 | 10 ± 5.0 |

| | | |
|---|---|---|
| a. The 0% human plasma assay contains 5% fetal bovine serum b. Number of independent replicates | | |

When tested against common HCV genotype 1 NS3 resistance-associated variants, such as V36M, R155K, D168A and D168V in GT 1a (H77), or T54A, R155K, D168V and V170A in GT 1b (Con-1), Compound 1 showed inhibitory activity nearly equivalent to that against wild-type HCV replicon. Compound 1 was also shown to have potent activity against many NS5A inhibitor and NS5B inhibitor resistance-associated variants *in vitro* (e.g., M28T, M28V, Q30D, Q30R, Y93C, Y93H, Y93N, L31V+Y93H, C316Y, M414T, Y448C, Y448H, S556G and S559G in GT 1a, and L28T, Y93H, S282T, C316Y, Y448H and S556G in GT lb).

## Claims

1. A combination of at least two direct acting antiviral agents (DAAs) for use in a method for treatment of hepatitis C virus infection, said method comprising administering to a patient in need thereof ribavirin and an effective amount of said combination of at least two DAAs, wherein said treatment does not include administration of interferon to said patient, and said treatment lasts for 12 or 16 weeks, and wherein said at least two DAAs comprises Compound 1, or a pharmaceutically acceptable salt thereof, and Compound 2, or a pharmaceutically acceptable salt thereof;
wherein the method also comprises administration of a DAA which is a HCV polymerase inhibitor, or a pharmaceutically acceptable salt thereof,
wherein Compound 1 is 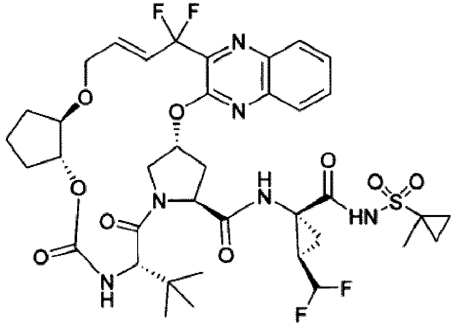 and wherein Compound 2 is

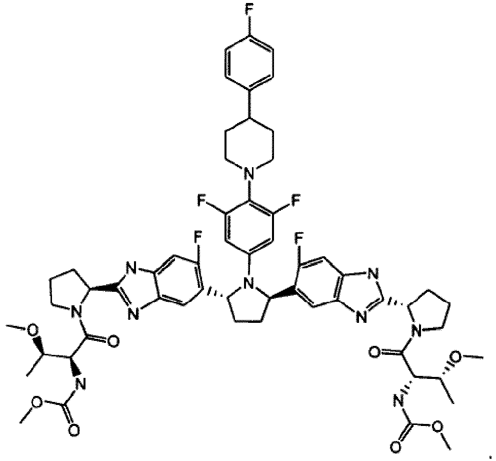

2. The combination of at least two DAAs for the use of claim 1, wherein said treatment lasts for 12 weeks.

3. The combination of at least two DAAs for the use of claim 1, wherein said treatment lasts for 16 weeks.

4. The combination of at least two DAAs for the use of any one of claims 1-3, wherein said patient is infected with hepatitis C virus genotype 1.

5. The combination of at least two DAAs for the use of any one of claims 1-3, wherein said patient is infected with hepatitis C virus genotype 1a.

6. The combination of at least two DAAs for the use of any one of claims 1-3, wherein said patient is infected with hepatitis C virus genotype 2.

7. The combination of at least two DAAs for the use of any one of claims 1-3, wherein said patient is infected with hepatitis C virus genotype 3.

8. The combination of at least two DAAs for the use of any one of claims 1-3, wherein said patient is infected with hepatitis C virus genotype 4.

9. The combination of at least two DAAs for the use of any one of claims 1-3, wherein said patient is infected with hepatitis C virus genotype 5.

10. The combination of at least two DAAs for the use of any one of claims 1-3, wherein said patient is infected with hepatitis C virus genotype 6.

11. The combination of at least two DAAs for the use of any one of claims 1-3, wherein said patient is without cirrhosis.

12. The combination of at least two DAAs for the use of any one of claims 1-3, wherein said patient is with compensated cirrhosis.

13. The combination of at least two DAAs for the use of any one of claims 1-3, wherein said patient is a treatment-naive patient.

14. The combination of at least two DAAs for the use of any one of claims 1-3, wherein said patient is an interferon non-responder.

15. The combination of at least two DAAs for the use of any one of claims 1-3, wherein said patient is a treatment-experienced patient.

16. The combination of at least two DAAs for the use of any one of claims 1-15, wherein said HCV polymerase inhibitor is a nucleotide or nucleoside polymerase inhibitor.

17. The combination of at least two DAAs for the use of any one of claims 1-15, wherein said HCV polymerase inhibitor is a non-nucleoside or non-nucleotide polymerase inhibitor.

18. The combination of at least two DAAs for the use of any one of claims 1-17, wherein Compound 1 or a pharmaceutically acceptable salt thereof, Compound 2 or a pharmaceutically acceptable salt thereof, and the HCV polymerase inhibitor or a pharmaceutically acceptable salt thereof, are administered once daily to said patient.

## Patentansprüche

1. Kombination aus zumindest zwei direkt wirkenden Antivirusmitteln (DAA) zur Verwendung bei einem Verfahren zur Behandlung einer Hepatitis-C-Virusinfektion, wobei das Verfahren die Verabreichung von Ribavirin und einer wirksamen Menge der Kombination aus zumindest zwei DAA an einen Patienten, der dessen bedarf, umfasst, wobei die Behandlung keine Verabreichung von Interferon an den Patienten umfasst und wobei die Behandlung 12 oder 16 Wochen lang dauert, und wobei die zumindest zwei DAA Verbindung 1 oder ein pharmazeutisch annehmbares Salz davon und Verbindung 2 oder ein pharmazeutisch annehmbares Salz davon umfassen;
wobei das Verfahren auch die Verabreichung eines DAAs, das ein HCV-Polymeraseinhibitor ist, oder eines pharmazeutisch annehmbaren Salzes davon umfasst,
wobei Verbindung 1 die folgende ist: 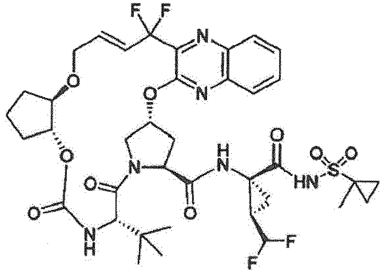 und wobei Verbindung 2 die folgende ist:

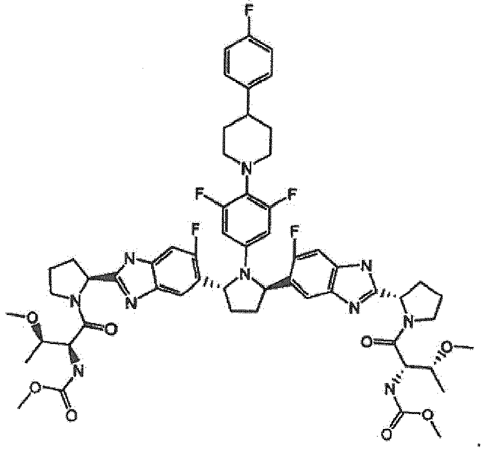

2. Kombination aus zumindest zwei DAA zur Verwendung nach Anspruch 1, wobei die Behandlung 12 Wochen lang dauert.

3. Kombination aus zumindest zwei DAA zur Verwendung nach Anspruch 1, wobei die Behandlung 16 Wochen lang dauert.

4. Kombination aus zumindest zwei DAA zur Verwendung nach einem der Ansprüche 1 bis 3, wobei der Patient mit Hepatitis-C-Virus-Genotyp 1 infiziert ist.

5. Kombination aus zumindest zwei DAA zur Verwendung nach einem der Ansprüche 1 bis 3, wobei der Patient mit Hepatitis-C-Virus-Genotyp 1a infiziert ist.

6. Kombination aus zumindest zwei DAA zur Verwendung nach einem der Ansprüche 1 bis 3, wobei der Patient mit Hepatitis-C-Virus-Genotyp 2 infiziert ist.

7. Kombination aus zumindest zwei DAA zur Verwendung nach einem der Ansprüche 1 bis 3, wobei der Patient mit Hepatitis-C-Virus-Genotyp 3 infiziert ist.

8. Kombination aus zumindest zwei DAA zur Verwendung nach einem der Ansprüche 1 bis 3, wobei der Patient mit Hepatitis-C-Virus-Genotyp 4 infiziert ist.

9. Kombination aus zumindest zwei DAA zur Verwendung nach einem der Ansprüche 1 bis 3, wobei der Patient mit Hepatitis-C-Virus-Genotyp 5 infiziert ist.

10. Kombination aus zumindest zwei DAA zur Verwendung nach einem der Ansprüche 1 bis 3, wobei der Patient mit Hepatitis-C-Virus-Genotyp 6 infiziert ist.

11. Kombination aus zumindest zwei DAA zur Verwendung nach einem der Ansprüche 1 bis 3, wobei der Patient keine Zirrhose aufweist.

12. Kombination aus zumindest zwei DAA zur Verwendung nach einem der Ansprüche 1 bis 3, wobei der Patient kompensierte Zirrhose aufweist.

13. Kombination aus zumindest zwei DAA zur Verwendung nach einem der Ansprüche 1 bis 3, wobei der Patient ein nicht vorbehandelter Patient ist.

14. Kombination aus zumindest zwei DAA zur Verwendung nach einem der Ansprüche 1 bis 3, wobei der Patient nicht auf Interferon anspricht.

15. Kombination aus zumindest zwei DAA zur Verwendung nach einem der Ansprüche 1 bis 3, wobei der Patient ein vorbehandelter Patient ist.

16. Kombination aus zumindest zwei DAA zur Verwendung nach einem der Ansprüche 1 bis 15, wobei der HCV-Polymeraseinhibitor ein Nucleotid- oder ein Nucleosid-Polymeraseinhibitor ist.

17. Kombination aus zumindest zwei DAA zur Verwendung nach einem der Ansprüche 1 bis 15, wobei der HCV-Polymeraseinhibitor ein Non-Nucleosid- oder ein Non-Nucleotid-Polymeraseinhibitor ist.

18. Kombination aus zumindest zwei DAA zur Verwendung nach einem der Ansprüche 1 bis 17, wobei Verbindung 1 oder ein pharmazeutisch annehmbares Salz davon, Verbindung 2 oder ein pharmazeutisch annehmbares Salz davon und der HCV-Polymeraseinhibitor oder ein pharmazeutisch annehmbares Salz davon einmal täglich an den Patienten verabreicht werden.

## Revendications

1. Combinaison d'au moins deux agents antiviraux à action directe (AAD) à utiliser dans une méthode de traitement d'une infection par le virus de l'hépatite C, ladite méthode comprenant l'administration à un patient en ayant besoin de ribavirine et d'une quantité efficace de ladite combinaison d'au moins deux AAD, dans laquelle ledit traitement ne comprend pas l'administration d'interféron audit patient, et ledit traitement dure 12 ou 16 semaines, et dans laquelle lesdits au moins deux AAD comprennent un composé 1, ou un sel pharmaceutiquement acceptable de celui-ci, et un composé 2, ou un sel pharmaceutiquement acceptable de celui-ci;
dans laquelle ladite méthode comprend également l'administration d'un DAA qui est un inhibiteur de polymérase de VHC, ou un sel pharmaceutiquement acceptable de celui-ci,
dans laquelle le composé 1 est 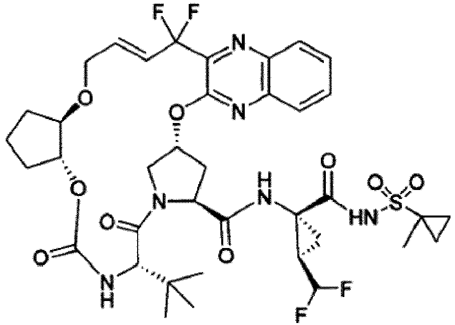 et dans laquelle le composé 2 est

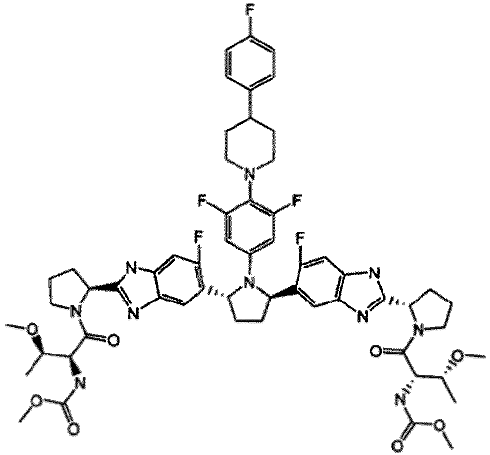

2. Combinaison d'au moins deux AAD pour l'utilisation selon la revendication 1, dans laquelle ledit traitement dure 12 semaines.

3. Combinaison d'au moins deux AAD pour l'utilisation selon la revendication 1, dans laquelle ledit traitement dure 16 semaines.

4. Combinaison d'au moins deux AAD pour l'utilisation de l'une quelconque des revendications 1 à 3, dans laquelle ledit patient est infecté par le génotype 1 du virus de l'hépatite C.

5. Combinaison d'au moins deux AAD pour l'utilisation de l'une quelconque des revendications 1 à 3, dans laquelle ledit patient est infecté par le génotype 1a du virus de l'hépatite C.

6. Combinaison d'au moins deux AAD pour l'utilisation de l'une quelconque des revendications 1 à 3, dans laquelle ledit patient est infecté par le génotype 2 du virus de l'hépatite C.

7. Combinaison d'au moins deux AAD pour l'utilisation de l'une quelconque des revendications 1 à 3, dans laquelle ledit patient est infecté par le génotype 3 du virus de l'hépatite C.

8. Combinaison d'au moins deux AAD pour l'utilisation de l'une quelconque des revendications 1 à 3, dans laquelle ledit patient est infecté par le génotype 4 du virus de l'hépatite C.

9. Combinaison d'au moins deux AAD pour l'utilisation de l'une quelconque des revendications 1 à 3, dans laquelle ledit patient est infecté par le génotype 5 du virus de l'hépatite C.

10. Combinaison d'au moins deux AAD pour l'utilisation de l'une quelconque des revendications 1 à 3, dans laquelle ledit patient est infecté par le génotype 6 du virus de l'hépatite C.

11. Combinaison d'au moins deux AAD pour l'utilisation de l'une quelconque des revendications 1 à 3, dans laquelle ledit patient ne présente pas de cirrhose.

12. Combinaison d'au moins deux AAD pour l'utilisation de l'une quelconque des revendications 1 à 3, dans laquelle ledit patient présente une cirrhose compensée.

13. Combinaison d'au moins deux AAD pour l'utilisation de l'une quelconque des revendications 1 à 3, dans laquelle ledit patient est un patient naïf de traitement.

14. Combinaison d'au moins deux AAD pour l'utilisation de l'une quelconque des revendications 1 à 3, dans laquelle ledit patient est un non-répondeur à l'interféron.

15. Combinaison d'au moins deux AAD pour l'utilisation de l'une quelconque des revendications 1 à 3, dans laquelle ledit patient est un patient ayant déjà reçu un traitement.

16. Combinaison d'au moins deux DAA pour l'utilisation de l'une quelconque des revendications 1 à 15, dans laquelle ledit inhibiteur de polymérase de VHC est un inhibiteur de polymérase nucléotidique ou nucléosidique.

17. Combinaison d'au moins deux DAA pour l'utilisation de l'une quelconque des revendications 1 à 15, dans laquelle ledit inhibiteur de polymérase de VHC est un inhibiteur de polymérase non nucléosidique ou non nucléotidique.

18. Combinaison d'au moins deux DAA pour l'utilisation de l'une quelconque des revendications 1 à 17, dans laquelle le composé 1 ou un sel pharmaceutiquement acceptable de celui-ci, le composé 2 ou un sel pharmaceutiquement acceptable de celui-ci, et l'inhibiteur de polymérase de VCH ou un sel pharmaceutiquement acceptable de celui-ci, sont administrés une fois par jour audit patient.
